Europäisches Patentamt

European Patent Office

Office européen des brevets

(11) Veröffentlichungsnummer : **0 108 073**
**B1**

(12) # EUROPÄISCHE PATENTSCHRIFT

(45) Veröffentlichungstag der Patentschrift :
**22.01.86**

(21) Anmeldenummer : **83900928.9**

(22) Anmeldetag : **29.03.83**

(86) Internationale Anmeldenummer :
**PCT/EP 83/00092**

(87) Internationale Veröffentlichungsnummer :
**WO/8303967 (24.11.83 Gazette 83/27)**

(51) Int. Cl.⁴ : **A 61 K 9/00**, A 61 M 31/00

(54) **CHIRURGISCHES HILFSMITTEL.**

(30) Priorität : **07.05.82 DE 3217109**

(43) Veröffentlichungstag der Anmeldung :
**16.05.84 Patentblatt 84/20**

(45) Bekanntmachung des Hinweises auf die Patenterteilung : **22.01.86 Patentblatt 86/04**

(84) Benannte Vertragsstaaten :
**AT BE CH DE FR GB LI NL SE**

(56) Entgegenhaltungen :
**DE-A- 2 320 373**
**DE-A- 2 917 037**
**DE-A- 3 037 270**

(73) Patentinhaber : **Merck Patent Gesellschaft mit beschränkter Haftung**
**Frankfurter Strasse 250**
**D-6100 Darmstadt (DE)**

(72) Erfinder : **HÄRLE, Anton**
**Drechslerweg 40**
**D-4400 Münster-Roxel (DE)**

## Beschreibung

Die Erfindung betrifft ein chirurgisches Hilfsmittel, das pharmazeutische Wirkstoffe in Trägermaterialien enthält, die eine protrahierte Freigabe der Wirkstoffe ermöglichen.

Aus der deutschen Patentschrift 23 20 373 sind Antibiotika-haltige Mittel bekanntgeworden, die als chirurgisches Material zur Bekämpfung von Infektionen eingesetzt werden. Sie dienen dabei beispielsweise zur Ausfüllung osteomyelitischer Höhlen und werden in Form von mit Hilfe von Fäden oder Drähten miteinander verbundenen Kugeln eingesetzt. Diese aus einem im Körper nicht resorbierbarem Material hergestellten Kugeln müssen nachträglich aus der Wunde operativ entfernt werden und sind daher bei der Versorgung von Weichteilinfektionen nicht vorteilhaft einsetzbar. Darüberhinaus ist eine gleichmäßige Verteilung der Kugeln über einen größeren Wundbereich nur sehr schwer erreichbar.

Aus der deutschen Patentschrift Nr. 29 17 037 ist es bekannt, arzneimittelhaltige partiell resorbierbare Mehrkomponentenmassen auf Basis von physiologisch verträglichen Stoffen einzusetzen, die als permanenter Knochenersatz dienen sollen und dabei voll in das biologische System integriert werden, ohne daß eine spätere erneute Operation zur Entfernung des Implantates erforderlich ist. Bei der Versorgung von normalen Wunden ist diese als Granulat oder Formmasse vorliegende Masse nicht einsetzbar, da hier keine Integration in den Knochen möglich ist.

Es bestand daher die Aufgabe, ein chirurgisches Hilfsmittel zu finden, das geeignet ist, medizinische Wirkstoffe aufzunehmen und gezielt, beispielsweise protrahiert freizugeben, und das insbesondere auch im normalen Wundbereich eingesetzt werden kann.

Die Aufgabe bestand weiterhin darin, das Mittel so zu gestalten, daß eine möglichst ungehinderte gewebliche Vereinigung der Wundflächen stattfinden kann ohne daß das eingesetzte chirurgische Hilfsmittel diese Wiedervereinigung stört oder später entfernt werden muß.

Schließlich war es auch Aufgabe, das Mittel so zu gestalten, daß es gestattet, medizinische Wirkstoffe an genau vorherbestimmbaren Orten, z. B. in gleichmäßiger Verteilung über den gesamten Wundbereich oder gezielt in einem oder mehreren Punkten lokalisiert zur Wirkung zu bringen.

Diese Aufgabe wurde durch die vorliegende Erfindung gelöst.

Gegenstand der Erfindung ist daher ein chirurgisches Hilfsmittel, enthaltend pharmazeutische Wirkstoffe in Trägermaterialien, die eine gezielte Freibage der Wirkstoffe ermöglichen, das dadurch gekennzeichnet ist, daß es in Form eines mit Freiräumen versehenen Flächengebildes gestaltet ist, wobei die einzelnen Freiräume eine Fläche von mindestens 0,1 cm$^2$ aufweisen.

Wesentlicher Vorteil der erfindungsgemäßen Hilfsmittel ist es, daß medizinische Wirkstoffe in definierten Abständen voneinander und damit in genau definierter räumlicher Verteilung in den Körper eingebracht werden können und dort an genau vorherbestimmbaren Orten ihre Wirkung entfalten können. Dabei wird durch die flächige Ausbildung und den hohen Anteil an Freiräumen eine direkte Kontaktierung der Wundflächen und damit eine problemlose Heilung ohne Ausbildung von Narbengewebe ermöglicht. Die Hilfsmittel können dabei z. B. in Form von Netzen oder Lochplatten ausgebildet sein, die ausreichend große Freiräume zur geweblichen Vereinigung der Wundflächen aufweisen.

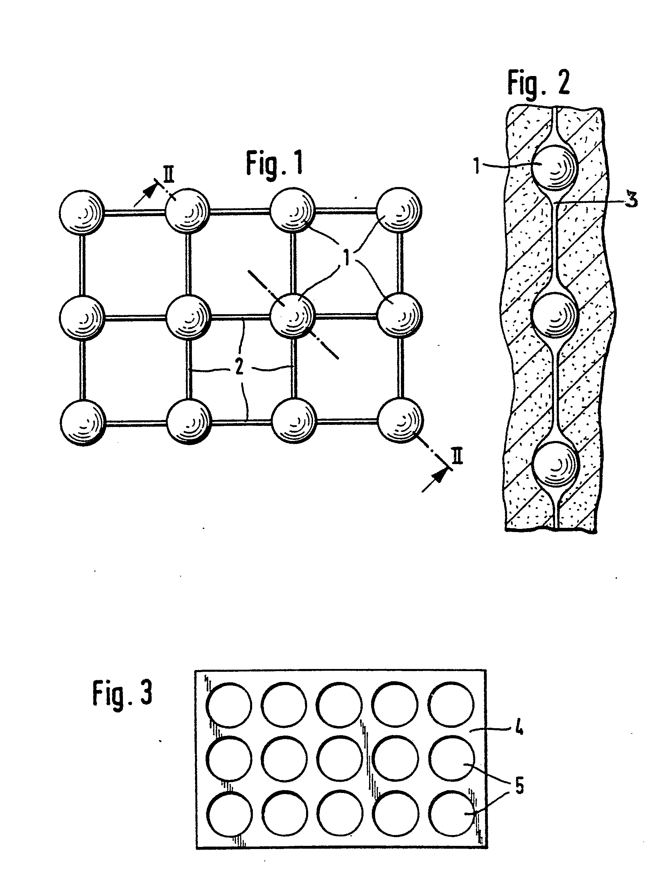

In den Zeichnungen sind einige bevorzugte Ausführungsformen der Erfindung dargestellt. Figur 1 zeigt eine Aufsicht auf ein Flächengebilde aus resorbierbaren Fäden (2), die ein Netz bilden, wobei an den Kreuzungspunkten (1) des Netzes die medizinisch wirksam werdenden Wirkstoffe angeordnet sind. Figur 2 zeigt einen Querschnitt entlang der Schnittlinie II-II durch ein in eine Wunde eingelegtes Netz der Figur 1.

Bei dieser Ausführungsform sind die das Netz bildenden Fäden (2) relativ dünn ausgebildet, und die wesentliche Masse ist in den Kreuzungspunkten lokalisiert. Dies hat den Vorteil, daß die Wundflächen (3) über wesentliche Bereiche der Wunde in direkten Kontakt kommen können. Die Wirkstoffe sind im wesentlichen in den Kreuzungspunkten (1) lokalisiert, von wo aus eine Diffusion in die umliegenden Freiräume erfolgt ; jedoch können auch die Fäden (2) Wirkstoffe enthalten.

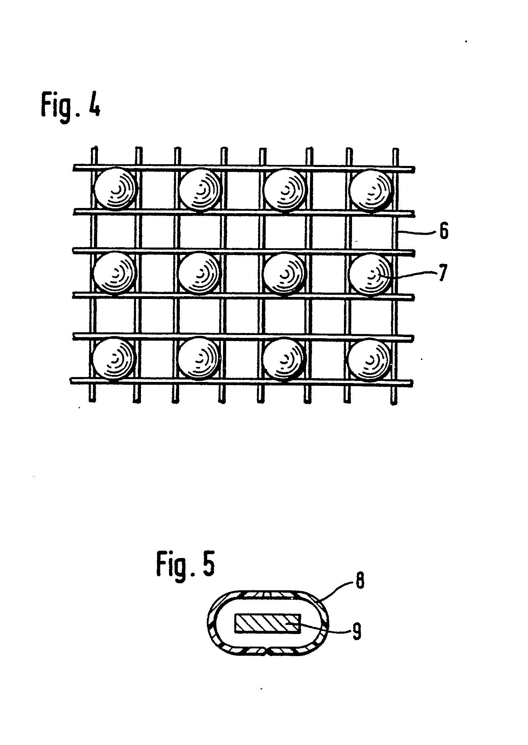

In Figur 3 ist ein Flächengebilde gezeigt, das als Platte (4) ausgebildet ist, die Freiräume (5) enthält. Dabei kann die Platte vorzugsweise insgesamt aus einem resorbierbarem Material bestehen, das die freizusetzenden Wirkstoffe enthält. Es ist jedoch im Prinzip auch möglich, einen Teil der Freiräume (5) mit wirkstoffhaltigen Formkörpern zu bestücken, während die freibleibenden Freiräume (5) eine Gewebeverbindung der beiden voneinander getrennten Wundschichten ermöglicht. Es können dabei z. B. die aus der deutschen Patentschrift 23 20 373 bekannten Kugeln eingesetzt werden, die gegebenenfalls untereinander mit Drähten oder Fäden verbunden sein können, so daß nach Resorption der Platte (4) das Ziehen der Kugeln in an sich bekannter Weise möglich ist. Durch die Anordnung der Kugeln in der Platte wird jedoch zum einen eine gleichmäßige Verteilung der Wirkstoffe im Wundbereich ermöglicht und zum anderen ein Verschlingen der Kugeln beim Ziehen vermieden.

In Figur 4 ist ein Flächengebilde gezeigt, das wiederum als Netz (6) ausgebildet ist. Dieses Netz kann zur Fixierung von wirkstoffhaltigen Formkörpern diesen, die hier als Kugeln (7) ausgebildet sind. Alternativ dazu oder ergänzend kann jedoch auch das Netz selbst aus wirkstoffhaltigem Material aufgebaut sein. Es ist dabei u. a. auch möglich, das Netz (6) und die Formkörper (7) mit unterschiedlichen Wirkstoffen zu bestücken. Durch die größere Beweglichkeit des Netzes (6) gegenüber der Platte (4) ist es möglich, die wirkstoffhaltigen Formkörper (7) in beliebiger räumlicher Verteilung, z. B. auch um einen Knochen oder Knochenstumpf herum zu fixieren.

In der Regel werden die erfindungsgemäßen Hilfsmittel aus einem körperverträglichen resorbierbarem Material gefertigt sein. Solche Materialien, die für andere chirurgische Zwecke schon vorgeschlagen wurden, sind in großer Zahl bekannt. Nur beispielhaft sollen hier genannt werden : Polyglykolide, Polylaktide, Polyhydroxycarbonsäuren, Polyaminosäuren und Kollagen. In bestimmten Fällen kann es jedoch auch notwendig sein, nicht resorbierbare Materialien einzusetzen. Dies ist z. B. dann der Fall, wenn die in den Flächengebilden enthaltenen Wirkstoffe nicht im Körper abgebaut werden, sondern wie z. B. Radioisotope nach einer bestimmten Wirkungsdauer wieder aus dem Körper entfernt werden müssen. Ein nicht auflösbares Netz kann relativ leicht wieder aus dem Körper entfernt werden, während der Verweildauer im Körper ist jedoch eine gute räumliche Verteilung der Wirkstoffe gewährleistet.

Beim Einsatz von z. B. Radioisotopen kann vorteilhaft auch ein in Figur 5 gezeigter Formkörper verwendet werden, der aus einer Kapsel (8) besteht, in die ein Radioisotop (9) eingelegt werden kann. Die Kapsel (8) ist aus einem nicht resorbierbarem Material, wie z. B. einem Acrylatpolymeren, in Form von Halbschalen gefertigt, die nach Einlegen des Strahlers verklebt oder verschweißt werden. Eine solche Kapsel (8) kann im Körper durch ein resorbierbares Netz entsprechend Figur 1 oder 4 oder eine resorbierbare Platte entsprechend Figur 3 gehalten werden. Durch das Einwachsen in die Wunde und das Resorbieren des Netzes wird sichergestellt, daß die Kapsel an dem gewünschten Platz angeordnet ist und bleibt, so daß die Strahlenbelastung genau kontrolliert werden kann und eine gewünschte örtliche Bestrahlung erzielbar ist, ohne daß befürchtet werden muß, daß bei Bewegungen des Patienten sich die Kugel verschieben kann. Sobald die Wundbereiche durch das Netz oder die Platte hindurch miteinander verwachsen sind, ist eine Verschiebung der Kapsel (8) nicht mehr möglich. Die in dem Netz oder der Platte enthaltenen z. B. antibakteriellen Wirkstoffe gewährleisten dabei eine problemlose Heilung der Wundbereiche.

Der Ausdruck « Wirkstoff » wird in einem sehr breiten Sinne gebraucht und umfaßt alle Artikel, die auf parenteralem Wege für die Heilung, Linderung, Behandlung und/oder Verhinderung von Gesundheitsstörungen bei Mensch und Tier vorgesehen sind oder die eine Funktion des Körpers von Mensch oder Tier zu beeinflussen vermögen.

Vor allem sind antibakterielle Wirkstoffe verschiedener Art, insbesondere Antibiotika, zu nennen. Ihr Wirkungsspektrum soll grampositive oder gramnegative Erreger oder vorzugsweise beide Gruppen umfassen. Möglichst sollen die Wirkstoffe bei den Erregern keine oder nur eine verzögerte Resistenz hervorrufen. Unter den antibakteriellen Wirkstoffen seien folgende Antibiotika beispielsweise herausgegriffen :

Aminoglykosid-Antibiotika wie Amikacin, Butirosin, Didesoxykanamycin B (DKB), Fortimycin, Gentamycin, Kanamycin, Lividomycin, Neomycin, Netilmicin, Ribostamycin, Sagamycine, Seldomycine und deren Epimere, Sisomicin, Sorbistin, Tobramycin, Streptomycine ; Lincomycine wie Clindamycin, Lincomycin und Rifamycine wie Rifampicin und Rifamycin.

Die Aminoglykosid-Antibiotika, insbesondere Gentamycin, sind dabei wegen ihres breiten antibakteriellen Spektrums besonders geeignet.

Es ist auch möglich, zwei oder mehrere dieser Antibiotika miteinander zu kombinieren, z. B. Gentamycin mit Clindamycin ; auch Kombinationen dieser Antibiotika mit anderen Wirkstoffen, z. B. mit Antiseptika, sind geeignet.

Bevorzugt sind auch andere Wirkstoffe, z. B. Antiseptika (wie Bromchlorophen, Hexetidin, Buclosamid, Salicylsäure, Cer-nitrat, Chlorhexidin, 5-Chlor-8-hydroxychinolin, Kupfer-8-hydroxy-chinolat, Acridinorange, Undecensäure, Undecoyliumchlorid, Silbersalze wie Silber-sulfadiazin, Magenid, Nitrofurazon, Cloflucarban, Tribromsalan, Taurolin, Noxythiolin), Tuberkulostatika wie z. B. Streptomycin, Rifamycine und Isonicotinsäurehydrazid, ferner Entzündungshemmer (wie Salicylate, Phenylbutazone, Indomethacin, Ibuprofen, p-Aminophenolderivate [z. B. Acetaminophen], Pyrazolone, Hydrocortisonpalmitat) sowie Cytostatika (wie Methotrexat, Fluorouracil, Vinblastin, Doxorubicin, Prednison). Gegebenenfalls können auch Wirkstoffe verschiedener Indikationsrichtungen miteinander kombiniert werden.

Auch die Art der Freisetzung der Wirkstoffe kann den Erfordernissen angepaßt werden. So ist es einmal möglich, durch Auswahl des Trägermaterials und der Konzentration des Wirkstoffs im Trägermaterial die Freisetzungsgeschwindigkeit zu steuern. Zum anderen kann auch gezielt die Löslichkeit des Wirkstoffs verringert werden und so eine stärkere Retardierung erreicht werden. Insbesondere für Antibiotika, aber auch für andere Wirkstoffgruppen kann dabei in vielen Fällen die Bildung schwer löslicher Salze vorgenommen werden. Für Aminoglykosid-Antibiotika besonders geeignet ist z. B. die Salzbildung mit Phosphorsäureestern der Hydroxyflavanoide.

Die Menge des zuzusetzenden Wirkstoffs kann in weiten Bereichen variiert werden und hängt im wesentlichen von seiner Aktivität ab. Im allgemeinen liegt die Menge des Wirkstoffes zwischen etwa 0,2-20 Gew.%, vorzugsweise bei etwa 5-15 Gew.% bezogen auf das chirurgische Hilfsmittel. Im Einzelfall ist jedoch darauf abzustellen, welche Gewebekonzentrationen zur wirksamen Beeinflussung der Gesundheitsstörung erforderlich sind.

Das erfindungsgemäße chirurgische Hilfsmittel schafft eine Fixierung für medizinisch wirksame Wirkstoffe direkt an dem Ort, wo eine therapeutische Notwendigkeit dafür gegeben ist. Gleichzeitig wird die gewünschte Dosierung ermöglicht, die bereits bei der Herstellung des Flächengebildes bestimmt wird und vom Operateur in Abhängigkeit vom jeweiligen Einsatzfall gewählt werden kann. Wesentlich ist, daß ausreichend große Freiräume im Flächengebilde vorliegen, die eine Gewebevereinigung der Wunde ermöglichen. So besteht der Anteil dieser Freiräume an der Gesamtfläche vorzugsweise mindestens 60 % gegebenenfalls sogar 75 % oder mehr. Dabei sollte jeder einzelne Freiraum eine Fläche von mindestens 0,1 cm² aufweisen, vorzugsweise mindestens 0,25 cm². Freiräume einer Größe von bis zu mehreren Quadratzentimetern sind jedoch auch realisierbar und gerade in Bezug auf die direkte Vereinigung der Wundflächen sehr vorteilhaft.

**Patentansprüche**

1. Chirurgisches Hilfsmittel, enthaltend pharmazeutische Wirkstoffe in Trägermaterialien, die eine gezielte Freigabe der Wirkstoffe ermöglichen, dadurch gekennzeichnet, daß das Hilfsmittel in Form eines mit Freiräumen versehenen Flächengebildes gestaltet ist, wobei die einzelnen Freiräume eine Fläche von mindestens 0,1 cm² aufweisen.

2. Chirurgisches Hilfsmittel nach Anspruch 1, dadurch gekennzeichnet, daß die Freiräume mindestens 60 % der Fläche des Flächengebildes ausmachen.

3. Chirurgisches Hilfsmittel nach Anspruch 1 oder 2, dadurch gekennzeichnet, daß es ein vom Körper resorbierbares Trägermaterial enthält.

4. Chirurgisches Hilfsmittel nach den Ansprüchen 1-3, dadurch gekennzeichnet, daß das Flächengebilde ein Netz bildet, wobei an den Kreuzungspunkten des Netzes die Wirkstoffe konzentriert vorliegen.

5. Chirurgisches Hilfsmittel nach den Ansprüchen 1-3, dadurch gekennzeichnet, daß auf gegebenenfalls wirkstofffreien Flächen wirkstoffhaltige Formkörper in definierten Abständen angeordnet sind.

6. Chirurgisches Hilfsmittel nach Anspruch 5, dadurch gekennzeichnet, daß die Formkörper aus Halbschalen zusammengesetzte Kugeln sind.

**Claims**

1. Surgical aid containing pharmaceutical active compounds in support materials which permit controlled release of the active compounds and which is characterised in that the aid is designed in the form of a flat article provided with free spaces, the individual free spacing having an area of at least 0.1 cm².

2. Surgical aid according to Claim 1, characterised in that the free spaces comprise at least 60 % of the area of the flat article.

3. Surgical aid according to Claim 1 or 2, characterised in that it contains support material which is absorbable by the body.

4. Surgical aid according to Claims 1-3, characterised in that the flat article forms a lattice, the active compounds being concentrated at the points of intersection of the lattice.

5. Surgical aid according to Claims 1-3, characterised in that moulded items containing active compound are located with defined spacings on flat areas which optionally contain no active compound.

6. Surgical aid according to Claim 5, characterised in that the moulded items are capsules constructed of half-shells.

**Revendications**

1. Accessoire chirurgical contenant des substances actives pharmaceutiques dans des matières-support, qui permettent une libération des substances actives selon le but visé, caractérisé en ce que l'accessoire est conçu sous la forme d'un élément plan comportant des espaces vides, chaque espace vide présentant une surface d'au moins 0,1 cm².

2. Accessoire chirurgical selon la revendication 1, caractérisé en ce que les espaces vides représentent au moins 60 % de la surface de l'élément plan.

3. Accessoire chirurgical selon la revendication 1 ou 2, caractérisé en ce qu'il contient une matière-support pouvant être résorbée par l'organisme.

4. Accessoire chirurgical selon les revendications 1-3, caractérisé en ce que l'élément plan forme une grille, les substances actives se trouvant concentrées aux points d'intersection de la grille.

5. Accessoire chirurgical selon les revendications 1-3, caractérisé en ce que sur les surfaces éventuellement exemptes de substances actives, des corps moulés contenant des substances actives sont disposés à des intervalles déterminés.

6. Accessoire chirurgical selon la revendication 5, caractérisé en ce que les corps moulés sont des billes constituées de demi-capsules.

Fig. 2

Fig. 1

Fig. 3

# Fig. 4

6

7

# Fig. 5

8

9